(19) Europäisches Patentamt
European Patent Office
Office européen des brevets

(11) **EP 2 314 330 A1**

(12) **EUROPÄISCHE PATENTANMELDUNG**

(43) Veröffentlichungstag:
27.04.2011 Patentblatt 2011/17

(51) Int Cl.:
*A61M 1/10* (2006.01)    *F16C 1/26* (2006.01)
*F16C 1/06* (2006.01)

(21) Anmeldenummer: 09075474.8

(22) Anmeldetag: 23.10.2009

(84) Benannte Vertragsstaaten:
AT BE BG CH CY CZ DE DK EE ES FI FR GB GR
HR HU IE IS IT LI LT LU LV MC MK MT NL NO PL
PT RO SE SI SK SM TR
Benannte Erstreckungsstaaten:
AL BA RS

(71) Anmelder: ECP Entwicklungsgesellschaft mbH
12247 Berlin (DE)

(72) Erfinder: Liebing, Reiner
14469 Potsdam (DE)

(74) Vertreter: Pfenning, Meinig & Partner GbR
Patent- und Rechtsanwälte
Joachimstaler Strasse 12
10719 Berlin (DE)

(54) **Flexible Wellenanordnung**

(57)    Gemäß der Erfindung ist eine flexible Wellen-anordnung (1) vorgesehen mit einer biegsamen Welle (5), einer Hülse (8), die die Welle umgibt, sowie einem Mantel (6), der die Welle und die Hülse umgibt. Die Hülse verläuft gemäß der Erfindung innerhalb des Mantels we-nigstens abschnittsweise bogenförmig und erzeugt damit stellenweise eine Anlage der Welle an der Innenkontur der Hülse. Hierdurch werden Schwingungen bzw. Ausschläge der Welle wirkungsvoll gedämpft.

Fig. 1

**Beschreibung**

Die Erfindung liegt auf dem Gebiet des Maschinenbaus, insbesondere der Mechanik, und beschäftigt sich mit der Übertragung von Drehmomenten und Bewegungen über flexible Wellen. Flexible Wellenantriebe sind aus verschiedenen Gebieten der Technik bekannt, wobei sie in Bezug auf die zu übertragenden Momente und die Drehzahlen stark variieren. Aus dem Gebiet flexibel anwendbarer Bearbeitungsmaschinen sind beispielsweise Wellenanordnungen bekannt, die mit relativ geringen Drehzahlen laufen und die hohe Momente übertragen sollen, während aus der zahnmedizinischen Technik Wellen bekannt sind, die mit extrem hohen Drehzahlen rotieren und geringere Drehmomente übertragen.

Beim Betrieb derartiger Wellen ergeben sich verschiedene Probleme, insbesondere infolge des Schwingungsverhaltens. Geringe Asymmetrien können an bestimmten Punkten einer Wellenanordnung und/oder bei bestimmten Drehzahlen zu Ausschlagbewegungen mit entsprechend unangenehmen Geräuschentwicklungen und zu mechanisch hohen Beanspruchungen durch Reibung zwischen der Welle und ihrer Führung führen.

Es sind verschiedene Vorschläge bekannt geworden, wie solche Ausschlagbewegungen verringert werden können, beispielsweise aus der deutschen Patentschrift Nr. 350682, in der das stellenweise Knicken einer Hülse einer flexiblen Welle vorgeschlagen wird, um abschnittsweise die Innenkontur der Hülse zu verengen und damit eine direkte und spielfreie Anlage der Welle in diesen Bereichen zu erzwingen, was mit einer gewissen Reibung verbunden ist, jedoch gegebenenfalls zu einer spielfreien Führung der Welle und damit zum Verhindern von Schlagbewegungen führen kann.

Aus der DE-OS 195 41 549 A1 ist bekannt, eine Hülse, in der die Welle läuft, gezielt nach innen radial auf die Welle hin zu verformen, um die Welle im Inneren der Hülse ausreichend festzulegen. Zudem ist dort erwähnt, dass entsprechende Deformationen abschnittsweise wendelförmig versetzt sein können. Es ist dort von Anquetschungen, Einwölbungen und Anstauchungen der Hülse die Rede.

Der vorliegenden Erfindung liegt im Hinblick auf den Stand der Technik die Aufgabe zugrunde, eine Wellenanordnung zu schaffen, die einen Betrieb der Welle mit hohen Drehzahlen bei geringer Reibung erlaubt, dabei sicherstellt, dass eine Mindeststandzeit der Wellenanordnung garantiert werden kann und dass Schwingungen der Welle zuverlässig verhindert werden. Dabei soll die Lösung insbesondere bei langen und sehr dünnen Wellen einsetzbar sein. Eine Verletzung oder Verformung der Welle selbst innerhalb der Wellenanordnung muss ausgeschlossen werden können.

Die Aufgabe wird gemäß der Erfindung mit den Merkmalen des Patentanspruchs 1 gelöst.

Im Gegensatz zum bekannten Stand der Technik werden gemäß der Erfindung für die Wellenanordnung eine biegsame Welle, eine diese umgebende Hülse und ein die Hülse und die Welle umgebender Mantel vorgesehen. Die Welle soll innerhalb des Mantels frei rotieren können, während der Mantel üblicherweise stillsteht.

Zur Lösung der Aufgabe ist vorgesehen, dass die Hülse innerhalb des Mantels wenigstens abschnittsweise bogenförmig verläuft. Dies bedeutet, dass die Hülse entlang der Länge des Mantels in einigen Bereichen einen anderen Abstand von einer Mittel-, Längs- oder Symmetrieachse des Mantels hat als in anderen Bereichen, oder , anders ausgedrückt, entlang der Länge des Mantels abschnittsweise, insbesondere in Bögen, von der Längsachse, Symmetrieachse oder Mittellinie des Mantels, die nicht notwendigerweise gerade verlaufen muß, abweicht.
Hierdurch wird erreicht, dass die Welle innerhalb der Hülse aufgrund ihrer Eigensteifigkeit abschnittsweise an der Innenwand der Hülse anliegt. Dadurch ergibt sich eine punktweise Festlegung der Welle in einem Gleitreibungsverhältnis an der Innenwand der Hülse. Eventuell auftretende Schwingungen der Welle werden somit mit minimaler Reibung gedämpft.

Diese Lösung hat den Vorteil, dass die Innenkontur der Hülse im Querschnitt weitestgehend unverändert bleiben kann, so dass die Welle innerhalb der Hülse den notwendigen Raum für die Rotation findet, beispielsweise in Form eines zylindrischen Innenraums, wenn die Hülse einen kreisrunden Querschnitt und hohlzylindrische Form aufweist. Die Bogenform der Hülse muss für diesen Zweck ausreichend ausgeprägt sein, was sich jedoch im individuellen Anwendungsfall leicht bewerkstelligen lässt.

In einer vorteilhaften Ausführungsform der Erfindung ist vorgesehen, dass zwischen der Innenkontur des Mantels und der Außenkontur der Hülse in wenigstens einem axial begrenzten Abschnitt an einem Teil des Umfangs wenigstens eine Abstandsanordnung vorgesehen ist. Dabei kann der Mantel im Wesentlichen gerade verlaufen, das heißt dass eventuelle Bögen, in denen der Mantel verläuft, wesentlich größeren Radius haben als die Bögen, die die Hülse innerhalb des Mantels beschreibt. Entsprechende Bogenradien des Mantels können beispielsweise mindestens 3-, 5-, 10- oder 50-mal so groß sein wie die Radien der Bogenabschnitte, die durch die Hülse innerhalb des Mantels gebildet werden. Üblicherweise liegt die Hülse innerhalb des Mantels mit radialem Spiel. Durch die Abstandseinrichtungen wird dieses Spiel eliminiert oder zumindest verringert, und die Hülse legt sich an die Innenkontur des Mantels auf einer Seite der Längsachse exzentrisch zu dieser an. Hierzu ist es notwendig, dass die entsprechende Abstandseinrichtung nicht azimutal am gesamten Umfang der Hülse oder des Mantels umläuft, sondern nur in einem bestimmten azimutalen Umfangsabschnitt vorgesehen ist.

Eine vorteilhafte Realisierung der Erfindung sieht vor, dass die Abstandsanordnung aus wenigstens einer Einprägung des Mantels radial nach innen oder der Hülse radial nach außen besteht.

Das Material der Hülse bzw. des Mantels kann derart dünn ausgebildet sein, dass Einprägungen die jeweilige Kontur, also die Innenkontur des Mantels und/oder die Außenkontur der Hülse, derartig einbuchten oder ausbuchten, dass mit der Prägung eine Erhebung geschaffen ist, die eine entsprechende Abstandseinrichtung bildet.

Zudem kann vorteilhaft vorgesehen sein, dass mehrere Abstandsanordnungen entlang der Länge der Wellenanordnung voneinander beabstandet vorgesehen sind. Durch eine derartige Anordnung der Abstandseinrichtungen wird eine wellenförmige Kontur der Hülse innerhalb des Mantels erzwungen, die beispielsweise mäanderartig oder sinuswellenförmig sein kann.

Es kann zudem vorgesehen sein, dass unmittelbar einander benachbarte Abstandsanordnungen am Umfang der Wellenanordnung azimutal in bezug auf die Welle winkelmäßig gegeneinander versetzt sind. Hierdurch ergibt sich ein dreidimensional wellenförmiger Verlauf der Hülse, so dass die Welle in mehreren Ebenen festgelegt wird.

Es kann dazu beispielsweise vorgesehen sein, dass die Abstandsanordnungen in axialer Richtung wendelförmig umlaufend angeordnet sind.

In einer besonders einfachen konstruktiven Verwirklichung der Erfindung kann vorgesehen sein, dass die Hülse als gewickelte Spirale ausgebildet ist. Damit ist eine einfache Herstellungsart der Hülse ermöglicht, und es ist zudem die Kühlung der Welle innerhalb der Hülse durch einen freien Austausch eines Kühlmediums, beispielsweise einer Flüssigkeit, innerhalb des Mantels durch die Spirale hindurch, erleichtert.

Vorteilhaft kann eine derartige Spirale aus einem Flachmaterial, beispielsweise in Form eines Bleches oder Kunststoffmaterials, bestehen. Dabei kann vorgesehen sein, dass die Abstandsanordnung als radial nach außen gerichtete Prägungen einzelner Windungen oder Gruppen von Windungen der Spirale ausgeführt sind. Es ist aber auch möglich, ein Rundmaterial zu verwenden und mit entsprechenden Prägungen zu versehen.

Der Vorteil einer solchen Konstruktion liegt darin, dass das vor dem Wickeln der Spirale gestreckte Material als solches geprägt werden kann und dass danach beim Wickeln der Spirale die entsprechenden Prägungen die gewünschten Erhebungen bilden. Dieses Vorgehen erleichtert die Fertigung, wobei die gewünschten Orte der Abstandseinrichtungen/Erhebungen durch entsprechende Abstände der Prägungen auf dem gestreckten Spiralmaterial vorbestimmt werden können. Die Prägungen können dabei jeweils nur auf einzelnen Windungen der Wicklungen oder auf einander unmittelbar benachbarten Windungen der Wicklung vorgesehen sein. Bei der Bildung von Gruppen von Prägungen liegen die Prägungen unmittelbar einander benachbarter Wicklungen der Spirale bezüglich des Umfangs nahe beieinander, und die Prägungen der jeweils nächsten Gruppe können dann winkelmäßig gegenüber der benachbarten Gruppe versetzt sein.

Es kann jedoch auch vorgesehen sein, dass einzelne Wicklungen in gewissen axialen Abständen zueinander in wendelförmig umlaufender Anordnung geprägt sind. Vorteilhaft kann zudem vorgesehen sein, dass die Prägungen oder Gruppen von Prägungen bezüglich des vor dem Wickeln gestreckten Spiralmaterials äquidistant zueinander angeordnet sind. Durch eine entsprechend berechnete Beabstandung der Prägungen auf dem Ausgangsmaterial der Spirale kann sichergestellt werden, dass auch der winkelmäßige Versatz aufeinanderfolgender Erhebungen/Abstandseinrichtungen regelmäßig wird.

Die Erfindung bezieht sich außer auf eine flexible Wellenanordnung gemäß der obigen Beschreibung auch auf ein Verfahren zur Herstellung einer solchen Wellenanordnung, bei dem ein Spiralmaterial vor dem Wickeln zu einer die Hülse bildenden Spirale derart geprägt wird, dass wenigstens eine radial bezüglich der Hülse nach außen gerichtete Erhebung entsteht.

Ein derartiges Herstellungsverfahren ist einerseits einfach auszuführen und erlaubt andererseits die genaue Vorherbestimmung der entsprechenden Abstandseinrichtungen, um den optimierten Verlauf der Hülse innerhalb des Mantels für einen störungslosen Betrieb der Welle zu gewährleisten.

Letztlich bezieht sich die Erfindung auch auf eine Blutpumpenanordnung mit einerflexiblen Wellenanordnung, wie sie oben in verschiedenen Ausführungsformen beschrieben ist. Derartige Blutpumpenanordnungen können beispielsweise minimalinvasiv zur Unterstützung oder zum Ersatz der Herztätigkeit durch ein Blutgefäß in den Körper eines Patienten eingeführt werden. Die Welle stellt dann die Antriebswelle eines Pumpenrotors dar und rotiert mit Drehzahlen von typisch mehr als 10 000 Upm.

Im Folgenden wird die Erfindung anhand eines Ausführungsbeispiels in einer Zeichnung gezeigt und anschließend beschrieben.

Dabei zeigt

Fig. 1    schematisch eine flexible Wellenanordnung mit einem Antrieb und einem mittels der Welle angetriebenen Aggregat,

Fig. 2    eine Wellenanordnung gemäß der Erfindung in einem ersten Querschnitt,

Fig. 3    eine Wellenanordnung gemäß der Erfindung in einem zweiten Querschnitt,

Fig. 4    die Hülse der erfindungsgemäßen Wellen- anordnung mit einer Welle in einer drei- dimensionalen Ansicht,

Fig. 5    eine Hülse gemäß der Erfindung in einer dreidimensionalen Ansicht,

Fig. 6    eine Hülse gemäß der Erfindung in einem Mantel in einem Längsschnitt,

Fig. 7    eine Wellenanordnung mit einer Welle, einer Hülse und einem Mantel gemäß der Erfindung in einem Längsschnitt, sowie

Fig. 8    ein Verteilungsschema der Prägungen auf dem Flachmaterial, aus dem später die Hülse ge- wickelt wird.

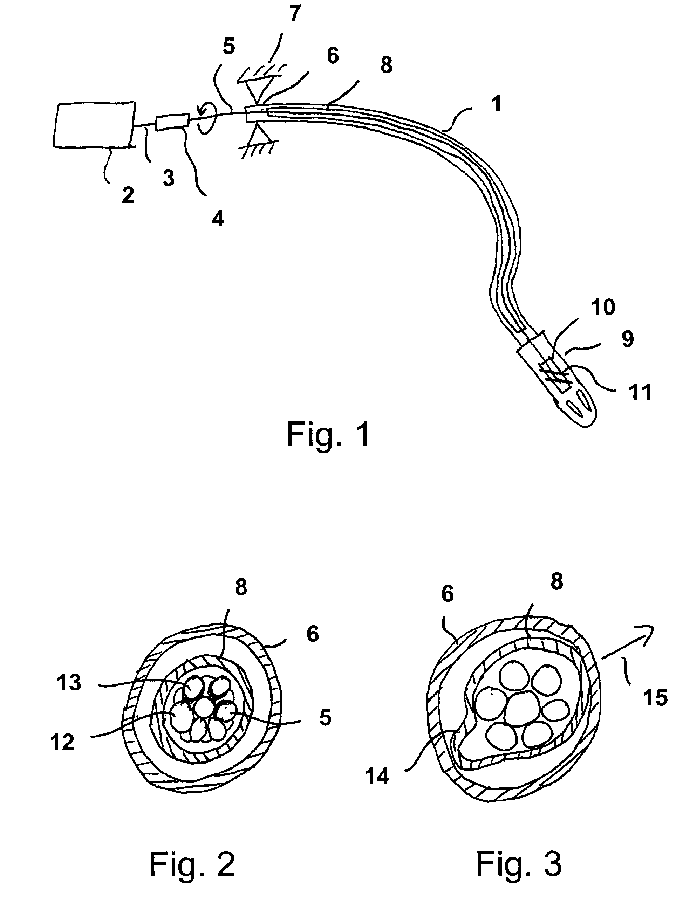

Fig. 1 zeigt die Wellenanordnung 1 in einer schematischen Ansicht mit einem Motorantrieb, der einen Elektromotor 2 sowie eine Motorwelle 3 und eine Kupplung 4 aufweist, mittels deren die flexible Welle 5 an dem Motor 2 drehfest angekoppelt ist. Zudem ist ein Mantel 6 der Wellenanordnung dargestellt, der motorseitig festgelegt ist in der Halterung 7. Im Inneren des Mantels ist eine Hülse 8 dargestellt, die die Welle 5 umgibt.

Auf der Abtriebsseite der Wellenanordnung ist eine Pumpe 9 mit einem Pumpenrotor 10 dargestellt, der Schaufelblätter 11 aufweist. Eine derartige Pumpe kann beispielsweise in der Medizintechnik im microinvasiven Bereich zur Förderung von Blut in einem Blutgefäß dienen und bildet eine sehr schnell drehende Axialpumpe.

Die erfindungsgemäße Wellenanordnung löst das Problem, dass die sehr schnell drehende, d. h. mit mehr als zehntausend Umdrehungen pro Minute rotierende Welle 5 zu Schlagbewegungen neigen kann, die zu störender Geräuschentwicklung und gegebenenfalls auch zu einem punktuell gesteigerten Abrieb führen können, so dass die Standzeit der Wellenanordnung beeinträchtigt ist.

Zur Lösung des Problems wird zunächst gemäß Fig. 2 der Querschnitt der Wellenanordnung betrachtet, bei dem außen der Mantel 6 dargestellt ist. Dieser kann beispielsweise als Kunststoffmantel oder auch als dünnwandiger Metallmantel ausgeführt sein. Innerhalb des Mantels 6 befindet sich die Hülse 8, die ebenfalls metallisch oder aus Kunststoff ausgeführt sein kann. Zwischen dem Mantel 6 und der Hülse 8 ist Spiel vorgesehen, da die Hülse 8 sich optional mit der Welle 5 innerhalb des Mantels bewegen kann.

Die Welle 5 besteht aus mehreren verseilten Strängen 12, 13, um die entsprechende Flexibilität zu erzeugen. Zwischen der Welle 5 und der Hülse 8 ist ebenfalls Spiel vorgesehen, um die Reibung zu minimieren.

Fig. 3 zeigt einen Querschnitt an einer Stelle der Wellenanordnung, an der eine Abstandseinrichtung zwischen der Hülse 8 und dem Mantel 6 vorgesehen ist. Die Abstandseinrichtung 14 ist als eine radial nach außen gerichtete Erhebung in der Hülse 8 ausgeführt. Durch diese Erhebung wird die Hülse 8 im Querschnitt in Richtung des Pfeils 15 innerhalb der Innenkontur des Mantels 6 gedrückt.

Da die Erhebung 14 in axialer Richtung nur in einem eng begrenzten Abschnitt der Wellenanordnung vorgesehen ist, wie weiter unten noch genauer ausgeführt wird, findet diese Auslenkung der Hülse 8 axial nur punktuell innerhalb des Mantels 6 statt, so dass die Hülse 8 eine Wellenform innerhalb des Mantels 6 einnimmt. Die Erhebung 14 kann beispielsweise durch eine Prägung im Material der Hülse 8 von innen radial nach außen erzeugt sein.

Die Welle ist beispielsweise aus dünnen Fasern/ Drähten durch Verdrillung gebildet, wobei jedes einzelne Verseilungselement der Welle einen Durchmesser aufweist, der zwischen der Hälfte und dem Doppelten der Höhe der Erhebung in der Hülse liegt. Damit wird ein Verhaken von Wellenelementen mit den Unregelmäßigkeiten/Prägungen der Hülse vermieden.

Zur Vermeidung des Verhakens der Wellenelemente in den Unregelmäßigkeiten/Prägungen der Hülse ist es in einer Abwandlung beispielsweise auch vorteilhaft, wenn die Prägungen in einer anderen Steigung um die Hülse ausgebildet bzw. angeordnet sind als die, in der die Wellenelemente der Welle gewickelt sind, so dass keine Parallelität zwischen Wellenelementen und den Unregelmäßigkeiten/Prägungen besteht.

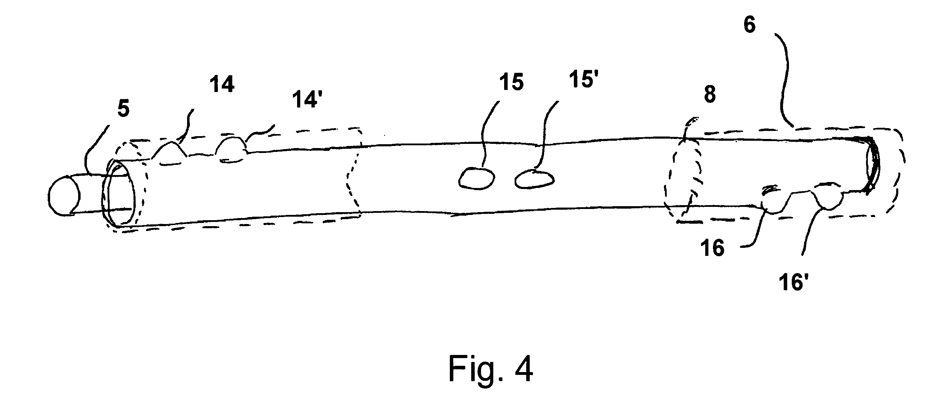

Fig. 4 zeigt in dreidimensionaler Ansicht mehrere Erhebungen 14, 14', 15, 15', 16, 16', wobei jeweils die Gruppen von Erhebungen axial voneinander beabstandet und azimutal winkelmäßig bezüglich der Längsachse der Wellenanordnung gegeneinander verschoben sind. Von links nach rechts durchlaufend ändert sich die Position der Gruppen von Erhebungen in Wendelform in Uhrzeigerrichtung umlaufend. Wird die dargestellte Hülse in einem langgestreckten Mantel 6 (gestrichelt dargestellt) befindlich gedacht, so wird die Hülse, so wie sie in der Fig. 4 dargestellt ist, im Bereich der Erhebungen 14, 14' gegenüber der Mantelachse nach unten verschoben, im Bereich der Erhebungen 15, 15' nach hinten in die Zeichenebene hinein und im Bereich der Erhebungen 16, 16' nach oben.

Es ergibt sich somit, wenn der Mantel als formstabil angesehen wird, eine Wellenform der Hülse 8. Diese führt dazu, dass die Welle 5, die aufgrund ihrer Eigensteifigkeit die Tendenz hat, ohne äußere Einwirkung gerade zu verlaufen, sich jeweils an die Innenkontur der Hülse 8 im Bereich der Erhebungen anlegt. Damit ist die Welle 5 ausreichend festgelegt, um über Reibung einen Großteil der Schlagbewegungen abzudämpfen.

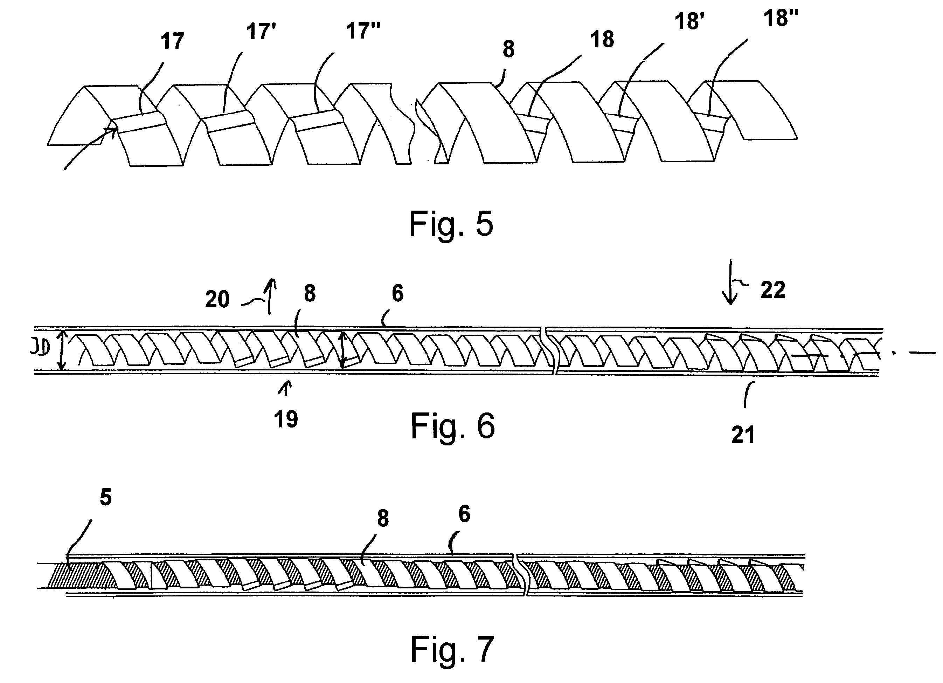

Fig. 5 zeigt in dreidimensionaler Darstellung ausschließlich eine Hülse 8, die in der dargestellten Ausführungsform als Spirale ausgebildet ist. Die Spirale weist Erhebungen 17, 17', 17'' und 18, 18', 18'' auf, die gegeneinander axial und in Umfangsrichtung versetzt sind. In dem dargestellten Beispiel sind die Erhebungen um 180 Grad am Umfang gegeneinander verschoben.

In der Fig. 6 ist gezeigt, auf welche Weise sich die dargestellte Spirale in einem Mantel 6 an dessen Innenkontur anlegt. Es ist dargestellt, dass im Bereich 19, in dem die Prägungen unterhalb der Mittelachse vorgesehen sind, die Hülse 8 nach oben in Richtung des Pfeils 20 verschoben ist, während die Hülse im Bereich 21 wegen der dort oberhalb der Mittelachse befindlichen Prägungen in Richtung des Pfeils 22 nach unten verschoben ist. Damit ergibt sich die angesprochene Wellenform der Hülse 8.

In der Fig. 7 ist die Figur weiter vervollständigt, indem auch eine Welle 5 eingezeichnet ist, die innerhalb der kreisrunden Kontur der Hülse geführt ist. Die Hülse 8 ist in der schon in Fig. 6 eingeführten Wellenform dargestellt, und die Welle 5 liegt zumindest punktuell an der Innenkontur der Hülse 8 unter Gleitreibungsbedingungen an.

Die Erfindung kann somit nutzbringend im Bereich der Medizintechnik eingesetzt werden, wo sehr schnell drehende Wellen innerhalb beispielsweise eines Hohlkatheters in einem Körper erforderlich sind. Der Hohlkatheter kann an sich auch die Rolle des Mantels übernehmen. Es kann aber auch die gesamte Wellenanordnung innerhalb eines Hohlkatheters angeordnet sein.

Es findet bei erfindungsgemäßer Gestaltung der Wellenanordnung eine Reduzierung der Reibung, des Verschleißes und der Geräuschentwicklung statt. Dies führt beispielsweise auch dazu, dass ein reduziertes Drehmoment zum Antrieb der Welle erforderlich ist, was insbesondere bei Verwendung komplexer Schleusen zu Durchführung der Welle vom Körperäußeren zum Körperinneren nützlich ist.

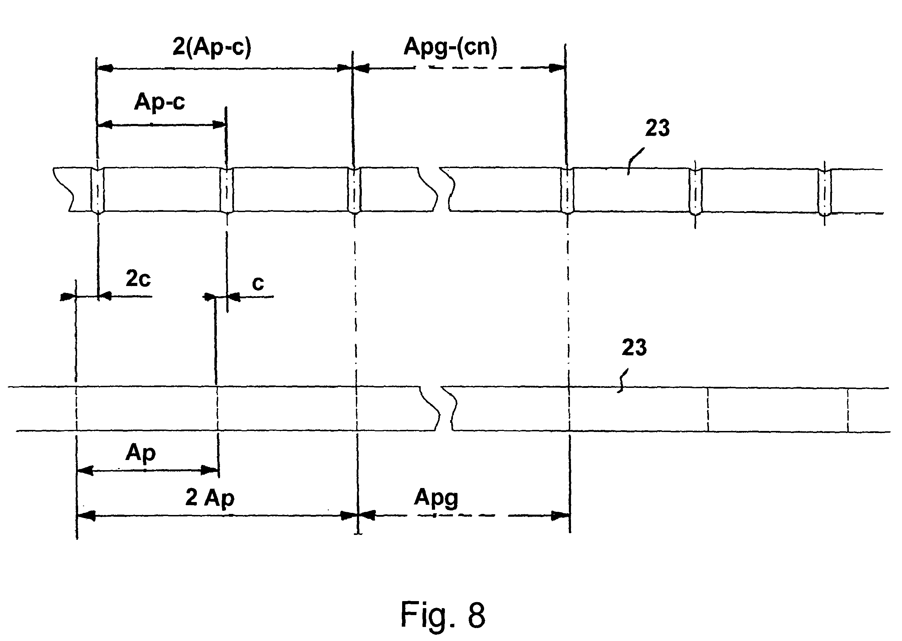

Fig. 8 zeigt ein Schema, das bei der Berechnung der Abstände der Prägungen in dem Ausgangsmaterial der Spirale nützlich ist. In der Figur ist das Strangmaterial der Spirale mit 23 bezeichnet. Im oberen Bereich der Fig. 8 ist der Strang vor dem Einbringen von Prägungen dargestellt, im unteren Bereich nach der Einbringung der Prägungen. Dabei ist mit Ap der Abstand der Prägungen innerhalb einer Prägegruppe bezeichnet. Dieser resultiert aus einer Näherungsgleichung für die Ellipsenberechnung, wobei der Betrag, um den sich die Länge durch die Prägung ändert, berücksichtigt ist. Der Abstand Ap zweier benachbarter Prägungen ergibt sich aus der Formel

$$AP \approx \left[ (a+b) \cdot \pi \cdot \left\{ 1 + \frac{3\lambda^2}{10 + \sqrt{4 - 3\lambda^2}} \right\} \right] + c \, ,$$

wobei

$$\lambda = \frac{a - b}{a + b}$$

gegeben ist. a bezeichnet den großen Halbkreis, b den kleinen Halbkreis der Ellipse, die durch die Kontur einer Windung der im Querschnitt betrachteten Spirale gegeben ist. Der Betrag c ergibt sich aus der Erweiterung des Prägeabstandes durch die Dehnung des Materials infolge der Prägung.

Es kann auch vorgesehen sein, dass anstelle des Parameters c der Parameter c $\pm$ x eingesetzt wird, um die Prägungen benachbarter Windungen am Umfang um einen konstanten Winkel gegeneinander zu versetzen und somit ein wendelförmiges Umlaufen der Prägestellen zu erreichen.

**Patentansprüche**

1. Flexible Wellenanordnung mit einer biegsamen Welle (5), einer diese umgebenden Hülse (8) und einem die Welle und die Hülse umgebenden Mantel (6), wobei die Hülse innerhalb des Mantels wenigstens abschnittsweise bogenförmig verläuft.

2. Flexible Wellenanordnung nach Anspruch 1, **dadurch gekennzeichnet, dass** zwischen der Innenkontur des Mantels (6) und der Außenkontur der Hülse (8) in wenigstens einem axial begrenzten Abschnitt auf einem Teil des Umfangs wenigstens eine Abstandsanordnung (14,14',15,15',16,16',17,17'17'',18,18',18'') vorgesehen ist.

3. Flexible Wellenanordnung nach Anspruch 2, **dadurch gekennzeichnet, dass** die Abstandsanordnung (14,14',15,15',16,16',17,17'17'',18,18',18'') aus wenigstens einer Einprägung des Mantels (6) radial nach innen oder der Hülse (8) radial nach außen besteht.

4. Flexible Wellenanordnung nach Anspruch 2 oder 3, **dadurch gekennzeichnet, dass** mehrere Abstandsanordnungen (14,14', 15,15',16,16',17,17'17'', 18,18',18'') entlang der Länge der Wellenanordnung voneinander beabstandet vorgesehen sind.

5. Flexible Wellenanordnung nach Anspruch 4, **dadurch gekennzeichnet, dass** unmittelbar einander benachbarte Abstandsanordnungen (14,14',15,15', 16,16',17,17'17'',18,18',18'') am Umfang der Wellenanordnung azimutal in bezug auf die Welle winkelmäßig gegeneinander versetzt sind.

6. Flexible Wellenanordnung nach Anspruch 5, **dadurch gekennzeichnet, dass** die Abstandsanordnungen (14,14', 15,15', 16,16',17,17'17'',18, 18',18'') in axialer Richtung wendelförmig umlaufend angeordnet sind.

7. Flexible Wellenanordnung nach Anspruch 1 oder einem der folgenden, **dadurch gekennzeichnet, dass** die Hülse (8) als gewickelte Spirale ausgebildet ist.

8. Flexible Wellenanordnung nach Anspruch 7, **dadurch gekennzeichnet, dass** die Spirale aus einem im wesentlichen flachen Material (23) besteht.

9. Flexible Wellenanordnung nach Anspruch 7 oder 8, **dadurch gekennzeichnet, dass** die Abstandsanordnung (14,14',15,15',16,16', 17,17'17'', 18,18',18'') als radial nach außen gerichtete Prägungen einzelner Windungen oder Gruppen von Windungen der Spirale ausgeführt sind.

10. Flexible Wellenanordnung nach Anspruch 9, **dadurch gekennzeichnet, dass** die Prägungen oder Gruppen von Prägungen bezüglich des vor dem Wickeln gestreckten Spiralmaterials äquidistant zueinander angeordnet sind.

11. Verfahren zur Herstellung einer Wellenanordnung nach einem der Ansprüche 1 bis 10, **dadurch gekennzeichnet, dass** ein Spiralmaterial vor dem Wickeln zu einer die Hülse (8) bildenden Spirale derart geprägt wird, dass wenigstens eine radial bezüglich der Hülse nach außen gerichtete Erhebung (14,14',15,15',16,16', 17,17'17'', 18,18',18'') entsteht.

12. Blutpumpenanordnung mit einer flexiblen Wellenanordnung gemäß einem der Ansprüche 1 bis 10, die mit einem Pumpenrotor verbunden ist.

Fig. 1

Fig. 2

Fig. 3

Fig. 4

**17** **17'** **17"** **8** **18** **18'** **18"**

Fig. 5

**20** **8** **6** **22**

**19** Fig. 6 **21**

**5** **8** **6**

Fig. 7

EP 2 314 330 A1

Fig. 8

EP 2 314 330 A1

Europäisches
Patentamt

European
Patent Office

Office européen
des brevets

## EUROPÄISCHER RECHERCHENBERICHT

Nummer der Anmeldung

EP 09 07 5474

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | JP 61 062614 A (NIPPON DENSO CO) 31. März 1986 (1986-03-31) * Abbildungen 1,7 * | 1 | INV. A61M1/10 F16C1/26 F16C1/06 |
| X | DE 103 24 717 A1 (AUDI NSU AUTO UNION AG [DE]) 4. August 2005 (2005-08-04) * Absatz [0002] * * Absatz [0030] * * Absatz [0037] - Absatz [0042] * * Abbildungen 5-8 * | 1,2,7 | |
| A | EP 0 415 333 A2 (NIPPON CABLE SYSTEM INC [JP]) 6. März 1991 (1991-03-06) * Seite 2, Zeile 5 * * Abbildungen 5-8 * | 1 | |
| X | US 2008/236124 A1 (HEINZELMANN GEORGE [DE]) 2. Oktober 2008 (2008-10-02) * Abbildungen 8-12 * | 1,2,4 | |
| Y,D | DE 350 682 C (ADAM SCHNEIDER A G) 25. März 1922 (1922-03-25) * Abbildungen 1-2 * | 1 | RECHERCHIERTE SACHGEBIETE (IPC) A61M F16C |
| Y | US 2005/151045 A1 (TORES DENIS [FR]) 14. Juli 2005 (2005-07-14) * Abbildung 3 * | 1 | |
| A | JP 62 188610 U (XXX) 1. Dezember 1987 (1987-12-01) * Abbildungen 1-4 * | 1 | |
| A | US 2006/155159 A1 (MELVIN DAVID B [US]) 13. Juli 2006 (2006-07-13) * Abbildungen 1a,1d * | 12 | |

-/--

Der vorliegende Recherchenbericht wurde für alle Patentansprüche erstellt

| Recherchenort | Abschlußdatum der Recherche | Prüfer |
|---|---|---|
| Berlin | 12. April 2010 | Wilson, Mark |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

**Europäisches Patentamt**
**European Patent Office**
**Office européen des brevets**

# EUROPÄISCHER RECHERCHENBERICHT

Nummer der Anmeldung

EP 09 07 5474

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2001/021831 A1 (FLEISCHHACKER MARK G [US] ET AL FLEISCHHACKER MARK G [US] ET AL) 13. September 2001 (2001-09-13) * Abbildungen 1-4 * ----- | 12 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Berlin | 12. April 2010 | Wilson, Mark |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
.......................................................
& : Mitglied der gleichen Patentfamilie, übereinstimmendes
   Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 09 07 5474

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-04-2010

| Im Recherchenbericht angeführtes Patentdokument | | Datum der Veröffentlichung | Mitglied(er) der Patentfamilie | | Datum der Veröffentlichung |
|---|---|---|---|---|---|
| JP 61062614 | A | 31-03-1986 | KEINE | | |
| DE 10324717 | A1 | 04-08-2005 | KEINE | | |
| EP 0415333 | A2 | 06-03-1991 | DE | 69008796 D1 | 16-06-1994 |
| | | | DE | 69008796 T2 | 22-12-1994 |
| US 2008236124 | A1 | 02-10-2008 | AT | 454032 T | 15-01-2010 |
| | | | CN | 101273685 A | 01-10-2008 |
| | | | DE | 102007015680 A1 | 23-10-2008 |
| | | | EP | 1974597 A1 | 01-10-2008 |
| | | | JP | 2008254168 A | 23-10-2008 |
| DE 350682 | C | 25-03-1922 | KEINE | | |
| US 2005151045 | A1 | 14-07-2005 | KEINE | | |
| JP 62188610 | U | 01-12-1987 | KEINE | | |
| US 2006155159 | A1 | 13-07-2006 | WO | 2004110257 A2 | 23-12-2004 |
| US 2001021831 | A1 | 13-09-2001 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 350682
- DE OS19541549 A1